# EUROPEAN PATENT SPECIFICATION

(11) **EP 1 364 624 B1**
(45) Date of publication and mention of the grant of the patent: **31.12.2008**
(21) Application number: 03253274.9
(22) Date of filing: 23.05.2003
(51) Int. Cl.: A61B 18/20

(54) **Improvements in laser treatment**
Verbesserung von Laserbehandlungen
Améliorations du traitement par laser

(30) Priority: 23.05.2002 US 382373 P
(43) Date of publication of application: 26.11.2003
(73) Proprietor: ED. GEISTLICH SÖHNE AG FÜR CHEMISCHE INDUSTRIE, 6110 Wolhusen (CH)
(72) Inventor: Strasser, Wolfgang, 79100 Freiburg (DE); Wokalek, Heinrich, 79100 Freiburg (DE)
(74) Representative: Marsden, John Christopher

(56) References cited:
- WO-A-02/03876
- US-A- 4 556 056
- US-A- 5 626 631
- PATENT ABSTRACTS OF JAPAN vol. 2000, no. 14, 5 March 2001 (2001-03-05) & JP 2000 316996 A (YA MAN LTD), 21 November 2000 (2000-11-21)

## Description

The present invention relates to laser treatment.

Lasers have been used in medical applications for decades. Lasers are particularly usefully in dermatological applications because of their ability to reduce scar tissue and improve the cosmetic appearance of the skin. For instance, photothermolysis laser (PTL) devices can provide up-to-date treatment available for dermatological applications such as the removal of vascular lesions, some benign pigmented lesions, tattoos and also for hair removal. However, a number of problems are associated with the use of PTL devices.

PTL devices work at high temperature, which naturally leads to unpleasant or painful sensations for subjects receiving treatment. As a result, compliance and the success rate of treatment are reduced, and the time involved in treatment is increased.

To attempt to solve this problem, cooling fans, ice packs or refrigeration units have used before applying the laser. However, the application of such cooling processes make treatment considerably more complicated. More recently, "shots" of liquid nitrogen have been directed at the skin shortly before the laser treatment is applied. These techniques are deficient because they are work-intensive and time-consuming, disrupt practice routine terribly, and are ultimately unpleasant, or even very painful, for the subjects (e.g. liquid nitrogen application).

Furthermore, it has been found that PTL treatment on previously cooled skin is less effective. A skin surface that has been deeply cooled before laser application reacts more slowly and less favorably to photothermolysis than a surface that has not been so deeply cooled. Thus, the energy densities often have to be increased to achieve the desired effect with the laser, which, in turn increases the pain the subject feels.

Effective PTL treatment requires transparency of the skin, which is a second problem. PTL utilizes different wavelengths of visible light, and in order to achieve the best effect in the target area of the skin (i.e. deep epidermis and corium), the skin surface must be as transparent as possible to permit the laser beam to penetrate down to the tissue to be treated. The higher the transparency, the lower the energy density needed for successful treatment, and the lower the damage that is caused to the surface epithelium, which should not be harmed. Only in these circumstances is laser treatment less traumatic and more selectively effective than the surgical alternative. The important structures in this respect are the vessels, pigment deposits in the epidermis and the superficial corium, hair follicles, and exogenous pigment (tattoos).

A third problem with PTL devices is that many PTL devices require continuous and direct contact with skin surface in order to achieve successful laser treatment without burning the skin. Abundant amounts of a semi-liquid ultrasound gel are usually applied to the skin to allow continuous and direct contact with the laser. Besides the unaesthetic appearance and the unpleasant sensation for the subject, in this case, laser treatment can literally degenerate into a slime bath. Also, therapists must take the greatest care to ensure sufficient gel remains under the laser head to maintain the contact technique. The constant movements of the applicator alone are sufficient to push the gel to one side so it has to be scooped up and put back under the applicator. This process is tedious, time-consuming, unprofessional and blocks the view of the area being treated. Such semi-liquid gels are considered a particular hindrance, not only to techniques for treating vascular changes, such as reticular varices and couperose, but also for pigmentation treatments, such as treating lentigenes and epilation for hirsutism. When semi-liquid gels are used, a smeary layer develops on the skin, which also detracts from the transparency and visibility during laser treatment.

Thus, there is a need for new and improved methods for performing PTL treatment, which are more efficient and less painful or uncomfortable for the subject.

The invention also provides use of a sheet-forming material in the manufacture of a disposable article which is a substantially clear sheet of solid or gel film for use in laser treatment of a subject, wherein said sheet is for application to an area of a surface of said subject prior to performing said laser treatment to said area in order to provide cooling of said surface during said treatment.

The surface to be treated can be any surface of a subject, such as a skin surface or an eye surface. In preferred embodiments, the invention involves treatment of a skin surface of a subject.

A skin surface area to be treated with a laser is covered with a substantially clear sheet of a film of hydrophilic organic transparent gel. If appropriate the sheet may be in the form of a strip. Preferably, the gel material is a transparent, clear film, e.g. having a thickness within the range of about 0.1-25 mm, preferably about 0.5-10 mm, and more preferably, within a range of about 1-5 mm.

The gel comprises a mixture of a hydrophilic polymer and at least one gellable substance of high molecular weight. Particularly preferred gel compositions and films are disclosed in U.S. Patent Nos. 4,556,056, 4,905,705 and 5,076,265, the entire contents of each of which are incorporated herein by reference. As an example, a commercially available polyacrylamide agar gel film called GELIPERM® is a suitable material. GELIPERM® is a trademark of and manufactured by Ed Geistlich Söhne AG für Chemische Industrie, Wolhusen, Switzerland, the present applicant.

A clear gel film is applied to a surface of skin to be subjected to laser treatment with a PTL device. Laser treatment is then applied to the skin through the film.

Further applications and advantages of the present invention are discussed below.

A clear film is applied to the skin prior to treatment so that the laser may be kept in constant contact with the area of the subject's skin that is to be treated. Lasers used for treatment may be commercially available PTL devices, such as an argon laser, Q-switched ruby laser, diode laser (Medio-Star, epilation and Dornier; starburst veins) and Aramis wrinkle laser. Acceptable films are gel compositions and films as disclosed in the afore-mentioned U.S. Patent Nos. 4,556,056, 4,905,705 and 5,076,265. However, one having ordinary skill in the art will understand that other films may be substituted having similar qualities and characteristics, as described herein.

The sheet may be cut or shaped to an appropriate size for covering the region of the skin to be treated, or may be pre-cut or pre-sized. The solid or gel film material is preferably a transparent, clear film, e.g. having a thickness within the range of about 0.1-25 mm, preferably about 0.5-10 mm, and more preferably, within a range of about 1-5 mm. The film may be disposable. Transparency is preferably optimized.

A commercially available product called Geliperm® has an extremely high transparency characteristic, and is therefore an exemplary preferred film. Contact between the laser applicator (e.g. PTL device) and the skin surface is easily maintained over any parts of the body, e.g. including the ala of the nose and the chin. Thus, treatment with PTL that may require cooling, transparency or skin contact for successful application can be applied problem-free to all parts of the body.

The application of the film prior to treatment allows for pain-free and long-lasting cooling of the skin. One novel result of the present invention is the reduction of the smell caused by heating structures containing keratin. Furthermore, heat erythema can be delayed. As a rule, heat erythema that usually develops rapidly, is delayed so long that a particular region can be treated with the laser without interruption. Erythema often means that small vessels cannot be treated because they "disappear" in the erythema. Thus, the delaying of erythema is particularly important when treating blood vessels.

According to an embodiment of the present invention, the film should also satisfy hygienic requirements. The film can be a disposable article and may be replaced several times during a single treatment to ensure ideal cooling, transparency and contact are always optimum.

The applicants performed significant testing that shows successful results. A transmission test using a transparent wound cover Geliperm®, polyacrylamide agar moist gel Plate 100x 30x3.3 mm in size, test beam profiles of 1Hz 44/Jcm², 1Hz 35/Jcm² and 1Hz 10/Jcm² were used with no discernible differences in the beam profile compared with final images without Geliperm®.

### Test transmission results:

| | | | | |
|---|---|---|---|---|
| without Geliperm® | 10.5 | with Geliperm® | 10.2 | |
| | J | | J | |
| | 10.3 | | 10.4 | |
| | J | | J | |
| | 10.5 | | 10.2 | |
| | J | | J | |
| | 10.4 | | 10.3 | Transmission: |
| | J | | J | 99% |
| | | | | |
| without Geliperm® | 43.5 | with Geliperm® | 43.5 | |
| | J | | J | |
| | 43.6 | | 43.5 | |
| | J | | J | |
| | 43.6 | | 43 J | |
| | J | | | |
| | 43.6 | | 43.3 | Transmission: |
| | | | J | 99% |

### Test for heating on uninterrupted operation:

Geliperm® was placed on a taper and fired at 4Hz 15 Jcm² for 20 minutes. After 20 minutes no discernable changes to Geliperm, no drying out, and no heating of the Geliperm®.

Test for burning hair: Tufts of hair were placed on a polytetrafluroethylene (PTFE) disc and covered with Geliperm®. A 12 mm hand piece was put on and the laser fired at 1Hz 44 J/cm². After 10 shots, Geliperm® had burned half-way through, at the same site there was no damage to the taper.

### Test for heat capacity (without skin contact):

Measurements were taken with a thermo-element and IR thermometer at room temperature 22°C. The results are shown in the table below:

| **t [min]** | **T Gelip. [°C]** | **Comment** |
|---|---|---|
| 0 | -3,5 | opaque and brittle |
| 2,5 | -3 | |
| 5 | -2,5 | |
| 10 | -2 | |
| 15 | -1 | |
| 20 | 0 | clear and soft |
| 25 | 2 | |
| 30 | 7,5 | |
| 35 | 12 | |
| 40 | 14 | |
| 45 | 15,5 | |
| 50 | 16 | |
| 55 | 16,5 | |
| 60 | 17 | |

### Test for heat capacity (with skin contact):

Measurements with IR thermometer were taken at room temperature 22°C. The results are shown in the table below:

| **t [sec]** | **T Gelip. [°C]** | **T skin [°C]** |
|---|---|---|
| 0 | 10 | 33 |
| 10 | 14 | |
| 20 | 17 | |
| 30 | 17 | |
| 40 | 18 | |
| 50 | 19,5 | |
| 60 | 20,5 | |
| 70 | 20,5 | |
| 80 | 21,5 | |
| 90 | 21,5 | |
| 100 | 22,5 | |
| 110 | 22,5 | |
| 120 | 23 | |
| 130 | 23 | 27 |

### Test for skin temperature:

Measurements were taken with an IR thermometer, and results are shown in the table below:

| **t [min]** | **T skin. [°C]** | **T. Gelip. [°C]** |
|---|---|---|
| 0 | 33 | 9 |
| 5 | 26 | |
| 10 | 25 | |
| 15 | 27 | |
| 20 | 26 | |
| 25 | 26 | 17 |

One skilled in the art will readily understand that the technique of the present invention considerably improves and simplifies the use of laser that work with either a contact gel or a cooling system, or for which cooling or better transparency is desirable, or the occasional production of the smell caused by heating structures containing keratin is to be avoided. Polyacrylamide agar moist gel moulds itself ideally to all surfaces, and causes no unpleasantness such as gel running off, thinning of the transmission layer with loss of the effect, or increasing pain. Treatment takes less time, is considerably more pleasant than with liquid gels for doctor and patient alike, is perfectly hygienic, requires no cleaning, and saves and protects the sensitive and expensive laser contact surfaces. During treatment, there is no attenuation to the laser beam as it passes through the clear film and hits the skin and structures being treated.

Geliperm® has been approved for medical application to the skin and wounds, and is available as a disposable article. All sites, extensive and small areas, can be treated automatically and quickly. The present invention is undoubtedly user-friendly and subject compliance. In tests, subjects evaluated the two techniques - liquid gel or the new product - and unequivocally chose the film over the liquid gel.

## Claims

1. Use of a sheet-forming material in the manufacture of a disposable article which is a substantially clear sheet of solid or gel film for use in laser treatment of a subject, wherein said sheet is for application to an area of a surface of said subject prior to performing said laser treatment to said area in order to provide cooling of said surface during said treatment.

2. Use as claimed in claim 1, wherein a sheet comprising a hydrophilic, organic transparent gel is manufactured.

3. Use as claimed in claim 1, wherein a sheet comprising a polyacrylamide agar moist gel is manufactured.

4. Use as claimed in any of the preceding claims, wherein a sheet having a thickness of 0.1-25 mm is manufactured.

5. Use as claimed in any of the preceding claims wherein a sheet having a thickness of 0.5-10 mm is manufactured.

6. Use as claimed in any of the preceding claims wherein a sheet having a thickness of 1-5 mm is manufactured.

## Patentansprüche

1. Verwendung eines flachmaterialbildenden Materials bei der Herstellung eines Einweg-Gegenstands, der ein im wesentlichen klares Flachmaterial aus einem festen Film oder einem Gelfilm zur Verwendung bei der Laserbehandlung eines Individuums ist, wobei das Flachmaterial zum Aufbringen auf einen Bereich einer Oberfläche des Individuums vor der Durchführung der Laserbehandlung dieses Bereichs, um während der Behandlung für eine Kühlung der Oberfläche zu sorgen, ist.

2. Verwendung wie in Anspruch 1 beansprucht, wobei ein Flachmaterial hergestellt wird, das ein hydrophiles, organisches transparentes Gel aufweist.

3. Verwendung wie in Anspruch 1 beansprucht; wobei ein Flachmaterial hergestellt wird, das ein feuchtes Polyacrylamid-Agar-Gel aufweist.

4. Verwendung wie in irgendeinem der vorangehenden Ansprüche beansprucht, wobei ein Flachmaterial mit einer Dicke von 0,1 bis 25 mm hergestellt wird.

5. Verwendung wie in irgendeinem der vorangehenden Ansprüche beansprucht, wobei ein Flachmaterial von einer Dicke von 0,5 bis 10 mm hergestellt wird.

6. Verwendung wie in irgendeinem der vorangehenden Ansprüche beansprucht, wobei ein Flachmaterial mit einer Dicke von 1 bis 5 mm hergestellt wird.

## Revendications

1. Utilisation d'un matériau de formation de feuille dans la fabrication d'un article jetable qui est une feuille pratiquement transparente en film solide ou en gel pour une utilisation dans le traitement au laser d'un sujet, dans laquelle ladite feuille est destinée à être appliquée à une zone d'une surface dudit sujet avant mise en oeuvre dudit traitement au laser sur ladite zone afin de permettre un refroidissement de ladite surface durant ledit traitement.

2. Utilisation selon la revendication 1, dans laquelle est fabriquée une feuille comprenant un gel transparent organique hydrophile.

3. Utilisation selon la revendication 1, dans laquelle est fabriquée une feuille comprenant un gel humide de polyacrylamide-gélose.

4. Utilisation selon l'une quelconque des revendications précédentes, dans laquelle est fabriquée une feuille ayant une épaisseur de 0,1 à 25 mm.

5. Utilisation selon l'une quelconque des revendications précédentes, dans laquelle est fabriquée une feuille ayant une épaisseur de 0,5 à 10 mm.

6. Utilisation selon l'une quelconque des revendications précédentes, dans laquelle est fabriquée une feuille ayant une épaisseur de 1 à 5 mm.
